# NEW EUROPEAN PATENT SPECIFICATION

(11) **EP 1 242 048 B2**
(45) Date of publication and mention of the opposition decision: **23.01.2019**
(45) Mention of the grant of the patent: 26.10.2005
(21) Application number: 00988840.5
(22) Date of filing: 28.12.2000
(51) Int. Cl.: A61K 9/12, A61K 9/14, A61K 9/72

(54) **INHALATION PARTICLES: method of preparation**
PARTIKEL ZUR INHALATION: Herstellungsmethode
PARTICULES POUR INHALATION: méthode de préparation

(30) Priority: 30.12.1999 FI 992820; 06.10.2000 FI 20002217
(43) Date of publication of application: 25.09.2002
(73) Proprietor: Orion Corporation, 02200 Espoo (FI)
(72) Inventor: WATANABE, Wiwik, FIN-02150 Espoo (FI); AHONEN, Petri, FIN-09630 Koisjärvi (FI); KAUPPINEN, Esko, FIN-00730 Helsinki (FI); JÄRVINEN, Raoul, FIN-02400 Kirkkonummi (FI); BROWN, David, FIN-00500 Helsinki (FI); JOKINIEMI, Jorma, FIN-01620 Vantaa (FI); MUTTONEN, Esa, FIN-02210 Espoo (FI)
(74) Representative: J A Kemp
(86) International application number: PCT/FI2000/001151
(87) International publication number: WO 2001/049263

(56) References cited:
- EP-A- 0 504 760
- US-A- 4 999 182
- US-A- 6 060 069
- HAJNALKA GOCZO ET AL: "Development of spherical crystals of acetylsalicylic acid for direct tablet-making" CHEM. PHARM BULL., vol. 48, no. 12, December 2000 (2000-12), pages 1877-1881, XP002901641

## Description

The present invention relates to methods for the preparation of inhalation particles suitable for pulmonary drug delivery.

### Background of the invention

Inhalation has become the primary route of administration in the treatment of asthma. This is because, besides providing direct access to the lungs, medication delivered through the respiratory tract provides rapid and predictable onset of action and requires lower dosages compared to the oral route.

There are three main delivery systems available for asthma therapy, namely the nebuliser, the pressurised metered-dose inhaler (pMDI) and the dry powder inhaler (DPI). The nebuliser is based on atomisation of liquid drug solutions using an external power source, while pMDIs and DPIs are based on suspension and dispersion of dry powder, respectively.

Dry powder inhalers (DPIs) are small, portable, cheap and user friendly as they do not require coordination between actuation and inspiration. Formulations for DPIs typically consist of fine drug particles admixed with an excipient of larger particle size, such as lactose, which facilitates the flowability of the powder. As DPIs generate aerosols without the use of propellants, they are considered more environmentally friendly than pMDIs. However, drawing a drug dose from the device into the lungs and aerosolising the drug particles to generate a 'respirable' particle size range, depends on air flow created from patient's inspiration. Consequently, a successful medication delivery via a DPI significantly depends on the patient inhalation techniques, which vary according to the patient's sex, age and disease state. More importantly, conventional powders used in DPIs often fails to provide a good delivery to the lungs and reproducible dosing over extended periods. Therefore, there is a need to improve the current dry powder system to provide dry powder which can be effectively aerosolised to maintain a uniform dose and which can be easily separated from the carrier materials, so as to generate respirable particles in the final dispersion.

Adhesion and detachment forces are important factors determining a successful delivery of powdered drug into the lungs by inhalation. The adhesion forces which include Van der Waals, capillary, Coulomb and electrical (double layers) forces, significantly influence powder flowability (and thus dose repeatability), aerosolisation of the powders and drug and carrier particle deagglomeration during delivery. It is well acknowledged that the adhesion and detachment behavior of particles is significantly dependent on particle size, shape, surface factors, electric charge and hygroscopicity. Therefore, the improved powder should have the following properties:
hydrophobic surface to eliminate capillary action;
sufficiently rough surface to increase the effective separation distance;
curved surface to reduce the contact areas;
uncharged surface and interior to reduce Coloumb and electrical forces; and
aerodynamic size of 1 to 5 µm to ensure an accurate delivery into the lungs.

Various techniques have been used to prepare drug powders for inhalation, including precipitation or crystallisation followed by drying and milling, spray-drying and supercritical fluid methods.

Precipitation or crystallisation followed by drying and size reduction process is a traditional method of drug powder preparation. The high energy milling operation generates particles, which are highly charged, and thus very cohesive. Furthermore, the milling process introduces surface and crystallographic damage, which raises concerns on the powder stability, and often results in particles with irregular fragments that could form strong aggregates. The milling process also generates flat faceted surfaces that contain many corner sites for condensation to occur, thus increasing adhesion forces and leading to inefficient drug-particle break-up. In addition, the multi-step processing causes a significant loss of materials during production as well as variability of product properties generated from different batches.

Spray-drying, on the other hand, is a one-step continuous process which can directly produce desirable particle size range and is commonly used to produce drug powders for inhalation delivery, see e.g. patent publications WO 96/32149, WO 97/41833, WO 97/44013, WO 98/31346 and WO 99/16419. The apparatus used in the spray drying method includes an atomizer or nozzle to spray the feed into a chamber where the feed is contacted to hot gas, and a particle collection system. The spray-drying process converts the liquid feed into dried particles by atomizing the feed to a spray form in a hot gaseous medium. Rapid evaporation of the droplets forms dried solid particles, which can be separated from the gas using a cyclone, an electrostatic precipitator, or a filter. The method is capable of controlling the particle size and size distribution, particle shape and particle density, by manipulating the process conditions. However, solid state properties (particularly crystallinity) can not be properly controlled. Consequently, the spray drying process produces amorphous particles, which have stability problems and a high tendency toward the moisture reabsorption, which is absolutely undesirable for pharmaceutical agents.

Techniques using supercritical fluids (SCFs) have also been explored for many years for particle production purposes. Similar to the spray-drying technique, this technique provides a direct formation of micron sized particles suitable for inhalation powders. The most commonly used supercritical fluid technologies for particle production are rapid expansion of supercritical solutions (RESS) and supercritical antisolvent (SAS) or gas antisolvent (GAS) methods. RESS is based on a rapid expansion of a SCF. The process involves dissolving the drug mixture in a SCF, followed by a rapid expansion of the fluid causing the compound to precipitate. This technique is capable of producing uniform particles, with control on the size distribution and morphology of particles. However, this technique is limited by the fact that most drugs have low solubility in the SCFs.

The second technique, namely SAS, is a recrystallisation process that relies on the capability of SCFs to act as an antisolvent to precipitate particles within a liquid solution. Unlike in the RESS technique, SAS does not require a high solubility of the drug compounds in the SCFs. Therefore, SAS is more commercially viable for powder production. However, various problems associated with control of particle size, product purity and solvent recovery have been reported.

Recently a solution enhanced dispersion by supercritical fluids (SEDS) was introduced, see e.g. patent publications GB 2322326, WO 95/01324, WO 95/01221, US 5851453 and WO 96/00610. This technique is based on simultaneous dispersion, solvent extraction and particle formation in a highly turbulent flow. SEDS is capable of generating uncharged and crystalline product, with the capability of controlling particle size and size distribution by manipulating process conditions. However, the particles formed have smooth surfaces and non-spherical shape, which cause inefficient break-up mechanism of the particles, and thus decrease the respirable fraction available for delivery to the lungs. Furthermore, the high energy cost and experimental difficulties (processes require sophisticated controller and high-pressure vessel formation) involved in the operation, make the process only attractive when all conventional techniques have failed. In addition, fulfilling environmental regulation is another problem of the technique.

EP-A-504760 describes pulmonary administration of ciclosporin in orthorhombic crystal form as well as crystalline ciclosporin in spheroidal particulate form.

US-A-4999182 describes stabilised zirconia, its process of preparation and application in ceramic compositions.

The present invention aims to provide a simple and efficient method which is able to produce consistent and controlled particle properties, including particle size and size distribution, shape, crystallinity, polymorphic phase, surface roughness and chemical purity. Such particles would be particularly well suited for drug delivery by inhalation.

### Summary of the Invention

It has been found that it is possible to obtain uncharged, spherical and crystalline particles incorporating one or several active ingredients, the particles having a narrow aerodynamic particle size distribution between about 1-5 µm, and which are useful in the preparation of compositions for dry powder inhalers, using an aerosol flow reactor method. Furthermore, it has been found that the method gives particles with rough surfaces. Rough surfaces are particularly desirable in inhalation particles since rough surface reduces the force required to break-up the aggregates of the particles or detach the particle from a coarse carrier. The particles exhibit improved dispersibility, good stability as a result of their crystalline nature, and more accurate delivery of the active agents into the targeted sites. Furthermore, the method of the invention provides a high purity product since the product purity only depends on the purity of solution precursors. Moreover, the methods and preparations are simple and can be easily scaled-up to higher production rates.

In one aspect the present invention provides a method for preparing particles suitable for pulmonary drug delivery, the method comprising the steps of:
providing a liquid feed stock comprising an active agent;
atomising the liquid feed stock to create droplets;
suspending said droplets in a carrier gas;
passing said carrier gas and droplets suspended therein through a heated tube flow reactor under predetermined residence time and temperature history; and
collecting the particles produced.

Particles suitable for use in pulmonary drug delivery by inhalation, which particles are obtainable by the above mentioned process, are spherical and crystalline, have a rough surface and incorporate one or several active agents. The mean mass aerodynamic diameter of the particles is typically between about 0.5 - 10 µm, more typically between about 1 - 5 µm. The aerodynamic particle size distribution of said particles is typically between about 0.5 - 10 µm, more typically between 1-5 µm.

An inhalation composition is described comprising spherical, crystalline particles obtainable by the process and incorporating one or several active agents. The particles may be formulated into an inhalation composition together with one or more pharmaceutically acceptable additives, diluents or carriers. Preferably, the composition is provided in the form of dry inhalation powder.

The method of the present invention differs significantly from the conventional spray-drying process. In spray-drying the hot gas is used as a source of heat to evaporate the solvent. The spray-drying chamber is only used as a place for the heat transfer to occur, the chamber itself is not heated. The temperature of the gas is changing across the chamber as heat transfer occurs between the cold feed and the hot gas. Furthermore, the evaporation is so rapid that it is not easy to properly control the temperature history and the residence time of each droplet and product particle. The crystallization can not be easily controlled either, and therefore the particles formed are commonly amorphous.

In the method of the present invention, the droplets are already suspended in the carrier gas before they are fed into the tubular flow reactor, which is placed in an oven set at a constant temperature. The carrier gas flows evenly in the tubular reactor with a constant rate, uniform temperature field and non-circulating flow. Therefore, the temperature history and the residence time of each droplet and product particle can be properly controlled and excellent uniformity of the particles can be ensured. Accordingly, the method provides better control of the droplet size distribution, and thus the particle size distribution. Furthermore, in contrast to spray drying, the method of the invention allows essentially complete crystallization of the particles.

### Brief Description of the Drawings

Figures 1a, 1b and 1c are schematic diagrams showing parts of the apparatus used in the method of the invention.
Figure 2 is a schematic diagram of the electrostatic precipitator.
Figures 3a and 3b show the normalised and cumulative mass size distribution of beclomethasone dipropionate particles produced by the method of the invention.
Figures 4a to 4d depict scanning electron microscopy images of the beclomethasone dipropionate powder produced by the method of the invention.

### Detailed Description of the Invention

The present invention employs an aerosol flow reactor method (aerosol synthesis method). It is a one-step continuous process, which can directly produce desirable particle size range. The method has been used to produce various materials, e.g. ceramic powder (US 5,061,682) or zirconia powder (US 4,999,182), at high operation temperatures. However, the method has not been used to produce pharmaceutical materials, which requires a significantly lower-temperature operation (less than 300 °C).

The method of the invention comprises generally the following steps:
a) providing a liquid feed stock comprising one or several active agents, b) atomising the liquid feed stock to create droplets, c) suspending said droplets in a carrier gas, d) passing said carrier gas and droplets suspended therein through a heated tube flow reactor under predetermined residence time and temperature history, and e) collecting the particles produced.

The liquid feed stock is prepared by mixing the active ingredient with a suitable liquid solution. The liquid feed stock may be in the form of a solution, suspension, dispersion, gel, emulsion, slurry or the like, and is preferably homogenous to ensure uniform distribution of the components in the mixture. The liquid feed stock in the form of a solution is preferred. In case of a combination of active ingredients is used, each active ingredients can be mixed with a suitable liquid solution, and the two or more liquid feed stocks are then mixed. It is also possible to mix all active ingredients directly in one liquid feed stock.

Various solvents may be employed in the preparation of the a liquid feed stock, including but not limited to, water, hydrocarbons, halogenated hydrocarbons, alchohols, ketones and the like. Examples of suitable solvents include water, hexane, perfluorohexane, ethanol, methanol, acetone, chloroform, methylene chloride and combinations thereof.

In case the liquid feed stock is a solution, the active agent or agents should be sufficiently soluble in the solvent so as to obtain, from the atomized droplets of the liquid feed stock, uniform particles with the desired particle size, size distribution, and, if several drugs are used, the drug ratio. The total solids dissolved may be present in wide range of concentrations, typically from about 0.1 % to about 10 % by weight, preferably from about 1 % to about 5 % by weight. A liquid feed stock containing relatively low concentration of solids results in particles having relatively small diameter. The finding of suitable liquid feed stock concentrations for each active agent/solvent combinations is considered to be a routine to one skilled in the art. Usually, the liquid feed stock concentration is firstly chosen at its maximum solubility so as to obtain the largest particle size with the atomizer and atomizer conditions used. From the results, a better approximation of the liquid feed stock concentration needed to obtain the desired particle size range with the atomizer and the atomizer conditions used can be easily achieved.

Any of a variety of active agents can be used in the method of the invention. The particles can be used to deliver locally or systemically a variety of active agents to a patient. Particularly suitable are agents which are typically used in the pulmonary delivery by inhalation, such as agents used in the treatment of asthma and other respiratory diseases. These include, but are not limited to, bronchodilators and steroidal anti-inflammatory drugs such as formoterol, salmeterol, salbutamol, budesonide, fluticasone propionate and beclomethasone dipropionate. Examples of other inhalable drugs include drugs for the treatment of respiratory disorders such as anticholinergic bronchodilators such as ipratropium bromide and the like, anti-allergic drugs such as nedocromil sodium, expectorants, mucolytics, antihistamines, cyclooxygenase inhibitors, leukotriene synthesis inhibitors, leukotriene antagonists, PLA2 inhibitors, PAF antagonists and prophylactics of asthma and combinations thereof. A particularly preferred combination is a combination of an anti-inflammatory agent and a bronchodilator.

Alternatively, the pharmaceutically active agent can be any of several types of inhalable, systemically active drugs including antiarrhythmic drugs, tranquilizers, cardiac glycosides, hormones, antihypertensive drugs, antidiabetic drugs, anticancer drugs, sedatives, analgesic drugs, antibiotics, antirheumatic drugs, immunotherapeutics, antifungal drugs, vaccines, antiviral drugs, proteins, peptides, vitamins and combinations thereof.

The liquid feed stock is atomized to create droplets in a suitable atomizer, which are well known in the art, such as a spray nozzle (e.g. a two fluid nozzle), an ultrasonic or air assisted nebuliser or a spinning disc, an ultrasonic nebulizer being preferred. Examples of the devices used in this process include ultrasonic generators sold under trademarks Omron NE-U12 and RBI Pyrosol 7901. While there are no special restrictions placed on the atomisers used in the process, it is recommended to use an atomiser, which can produce uniform droplets of constant composition and in a specific size range. Such devices are suitable to produce dry powders of controlled composition and with particle size range suitable for dry powder inhalation.

The droplets of the liquid feed stock are suspended in a carrier gas before passing through a heated tube flow reactor. The carrier gas must be inert with respect to the drug molecules and the solvent. It is recommended to use nitrogen gas or other inert gases. The temperature of the carrier gas is typically ambient. To maintain a uniform solution concentration in the droplets in the suspending phase, it is preferred to bubble the carrier gas through a bottle containing the same solvent as the liquid feed stock before entering the atomizer.

Because the droplets are already suspended in the carrier gas when fed into the reactor (i.e. the droplet generation and flow reactor are separated), the temperature history and residence time of each droplet and product particle can be better controlled than in the conventional spray drying method. Therefore, excellent uniformity of the resulted particles and narrow particle size distribution can be ensured.

The droplets suspended in the carrier gas are passed through a tubular flow reactor, which is maintained at a constant temperature. The temperature and the flow rate of the carrier gas are adjusted to evaporate the solvent and to allow the crystallisation process to complete. The particles formed are then collected using an electrostatic precipitator, a cyclone, a planar filter (e.g. nylon) or other particle collecting devices.

The aerosol flow reactor conditions are selected such that crystalline spherical particles of homogeneous constituents having a narrow particle size distribution and rough surfaces are formed. The mean mass aerodynamic diameter of the particles is between about 0.5-10 µm, preferably between about 1-5 µm. Particularly it is preferred that more than 98 % of the mass is in particles having a diameter of 5 µm or less, and less than about 5 % of the mass being in particles having a diameter of 0.5 µm or less. It is particularly preferred that the aerodynamic particle size distribution of said particles is between about 0.5-10 µm, more preferably between about 1-5 µm.

The particles are in crystalline form, i.e. the relative degree of crystallinity preferably 90 % or higher, more preferably 95 % or higher, most preferably 99 % or higher. The relative degree of crystallinity can be determined based on the x-ray powder diffraction patterns. The value of the relative degree of crystallinity can be estimated by a known method of broadening of the diffraction maxima (FWHM-values).

The particles are essentially spherical, i.e. the spherical form is consistent and apparent when examined under a scanning electron microscope. The spherical form reduces the contact areas between particles and thereby improves aerosolization and deagglomeration of the particles upon inhalation.

Generally, the surface of the spherical particles is rough, i.e. the roughness is consistent over the entire surface of the particle, apparent when examined under the scanning electron microscope, and the ratio of the maximum and minimum diameter of the particle is between 1.001 - 1.5. Rough surface is advantageous since it increases the effective separation distance of the particles again improving aerosolization and deagglomeration properties of the particles.

If desired, various additives known in the art may be additionally incorporated in the particles together with the active ingredients. Such additives include e.g. diluents such as lactose, carriers and stabilizers and the like. In such case the additives are included in the liquid feed stock of the process together with the active ingredients. Also such additives incorporated in the particle are preferably in crystalline form. It is particularly preferred that at least about 90 w-% of the total weight of the particle is in crystalline form.

However, in order to reduce the amount of material other than the active ingredients potentially reaching the lungs, it is preferred that the active ingredients constitute at least 90 w-%, preferably at least 95 w-%, more preferably at least 99 w-%, of the total weight of particles. Most preferably the particles are free from other material than the active ingredients.

The particle size may be controlled to any expected particle size ranges by selection of the atomizer and concentration of the liquid feed stock, or employing a droplet size modification apparatus (e.g. impactor or virtual impactor, or using size selective collection of particles, e.g. cyclone) downstream of the flow reactor. Normally, however, this is not needed.

For the tubular flow reactor, while there are no particular restrictions, it is recommended to use a vertical, rather than horizontal configuration in order to minimise buoyancy effects and related losses due to recirculating flow. To insure uniform temperature and flow fields in the hot zone of the reactor, CFD (Computational Fluid Dynamics) calculations have shown that it is preferable that the aerosol flows against gravity. Flow in any other direction tends to produce undesirable reactor conditions. The reactor tube is preferably placed inside an oven to maintain a uniform reactor wall temperature during the process. The oven can be of any kind, which has sufficient temperature control (i.e. ± 1 °C or less) at low temperatures (less than 300 °C). The temperature of the oven is set such that the materials being processed do not decompose. Typically the selected oven temperature is within the range of about 30 to 300 °C, more typically between about 70 to 200 °C. For example, since the melting point of beclomethasone dipropionate is about 210°C, the range of oven temperature used for beclomethasone dipropionate particle production may vary between 30 to 200 °C, preferably between 70 to 150 °C.

While there are no particular restrictions placed on the particle collection, it is recommended to use a system, which can be heated to prevent the re-condensation process. Electrostatic precipitators, cyclones and/or filters can be used for this purpose. Accordingly, the particle collection system and the line from the flow reactor outlet to the particle collection system are preferably heated to a temperature above the boiling point of the solution to prevent the re-condensation process to occur. However, the temperature should not be too high so as to cause material degradation. For example, for beclomethasone dipropionate dissolved in ethanol, the temperature of the collection system and the line may be kept constant at a temperature between 80 to 150 °C, preferably between 100 and 120 °C.

The particles may be formulated into an inhalation composition together with one or more pharmaceutically acceptable additives, diluents or carriers. Examples of suitable solid diluents or carriers comprise lactose, dextran, mannitol and glucose, lactose being preferred. Examples of aerosol carriers include non-chlorofluorocarbon-based carriers such as HFA (hydrofluoroalkane). The use of aqueous carriers is also possible. Typical additives include solubilizers, stabilizers, flavouring agents, colorizing agents and preserving agents.

The particles are preferably administered in the form of a dry powder composition. The particles obtained are generally in the form of individual (unagglomerated) particles which are well suited for pulmonary drug delivery by inhalation as such, e.g. they can be filled directly into capsules, cartridges, blister packs or reservoirs of dry powder inhalers. However, if desired the particles may be adapted to form loose agglomerates of several individual particles, said agglomerates breaking into individual particles upon dispersion in the inhaled air stream. The particles may also be combined with pharmaceutically acceptable carrier materials or excipients typically used in dry inhalation powders. Such carriers may be used simply as bulking agents or to improve the dispersibility of the powder. For example, the particles may be used in admixture with carrier particles, e.g. lactose, having larger particle size than the active ingredients, typically in the range of 5 to 100 µm. If the composition contains a carrier, the total amount of the active ingredients is typically about 0.1 - 50 % (w/w), preferably about 1 - 10 % (w/w), based on total weight of the composition. Such compositions can be prepared by methods known in the art.

The particles can be also administered in the form of pressurized metered dose inhalation suspension, where the particles are suspended in pressurized aerosol carrier and delivered using pressurized metered dose inhaler (pMDI).

The invention is further illustrated by the following experiments, which are not meant to limit the scope of the invention.

### Experiments

All compositions produced according to the present invention fulfill the strict specification for content and purity required for pharmaceutical products.

### Example 1. Inhalation particles comprising beclomethasone dipropionate, polymorphic form I

### Preparation of the liquid feed stock

Beclomethasone dipropionate is a glucocorticoid, which is practically insoluble in water, freely soluble in acetone and in chloroform, and sparingly soluble in alcohol. Thus, the solvent could be acetone, chloroform, methanol, ethanol, or other alcohols. In the current experiments, ethanol was used as a solvent, not only because ethanol is cheap and readily available but it is also recommended for use in production of pharmaceutical agents because it is non-toxic.

The beclomethasone dipropionate liquid feed stock was prepared by dissolving 1 gram of beclomethasone dipropionate powder in 40 ml of ethanol (99.5%) at room temperature.

### Aerosol Synthesis

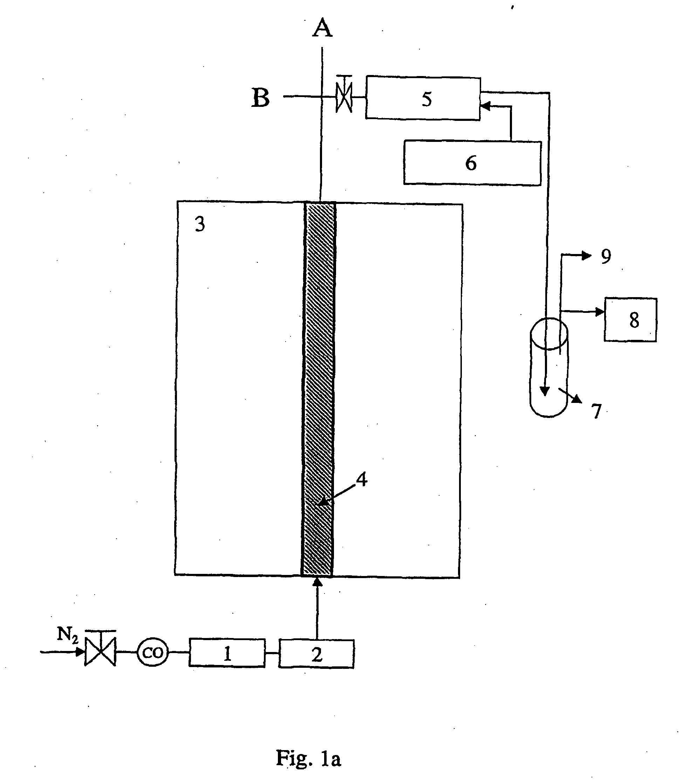

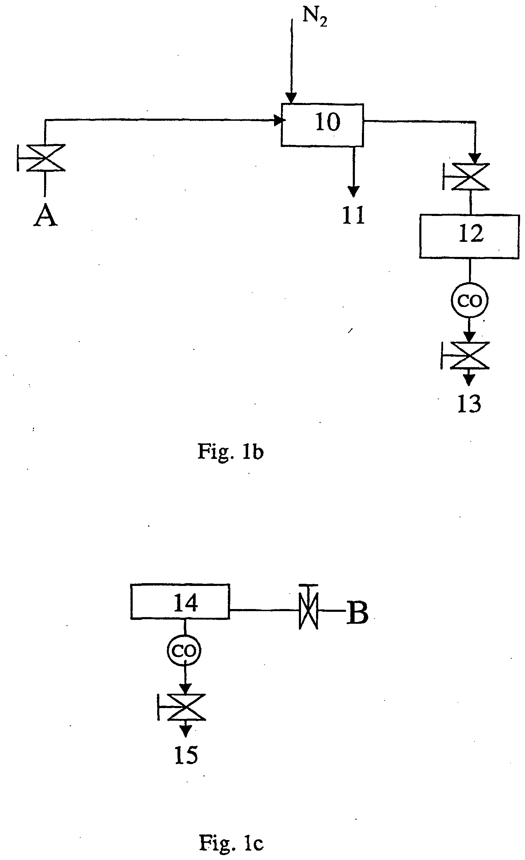

Figure 1a shows the experimental set-up of the particle synthesis, and Fig. 1b and 1c show optional configurations used for particle analysis. The liquid feed stock described above was atomised using an ultrasonic atomizer (2), sold under trade mark RBI Pyrosol 7901. The resulted droplets, which were suspended into a carrier gas, were then passed through a heated tube flow reactor (4). Nitrogen gas was used as a carrier gas, with a constant flow rate of 1.5 l/min. To maintain a uniform solution concentration in the atomizer, the carrier gas was bubbled through ethanol in a saturation bottle (1) before entering the atomizer. A vertical tube, which was inserted into an oven (3), was used to dry up the droplets. The oven used was a WTB Binder FD/FED 400, which has temperature variations of ± 1 and ± 2°C for temperature at 70 and 150°C, respectively. The tube was made of stainless steel, with an inner diameter and a heated length of 29.7 and 800 mm, respectively. The oven temperature was set at 150 °C. The minimum particle residence time in the heated zone under the selected process conditions was approximately 12 seconds. From the CFD calculation, it is shown that temperature field is uniform and the velocity is fully developed and non-circulating in the heated zone.

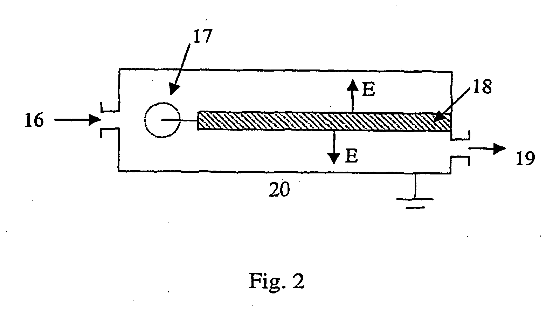

The resulted particles were then collected using an electrostatic precipitator (ESP) (5) connected to a high voltage generator (6). The exhaust gas was led from ESP via a dripping bottle (7) to exit (9). Figure 2 shows the schematic diagram of ESP having inlet (16) and exit for exhaust gas (19). The ESP was made of a tubular stainless steel collection plate (20) with inside diameter and length of 10 and 50 cm, respectively. A 0.05 mm diameter tungsten wire was placed on the center axis of the collection plate and a high voltage (18) of 16 kV was applied between the wire and the plate. The high electric field formed a corona discharge (17) on the wire and charged the gas molecules. The gas ions were then formed. These ions migrated across the space between the wire and the plate under the influence of the applied electric field. During the migration, the ions collided with the aerosol particles, which thus acquired charge. The charged particles then migrated toward the grounded surface electrode. When the particles struck the grounded plate, they lost their charges and adhered to the plate surface via surface forces. Therefore, the particles collected were not charged. Temperature in the ESP and in the line from tubular tube outlet to the ESP, were maintained at a constant temperature of 100 °C to avoid condensation of organic vapours and moisture to occur. Condensation particle counter (CPC) model 3022, shown as (8) in Fig. 1a, was used to determine efficiency of the ESP. Particles collected were then removed from the plate surface of ESP by scraping, and then placed in a tight glass bottle to avoid moisture penetration or other contamination.

### Characterisation

### i. Particle size analysis

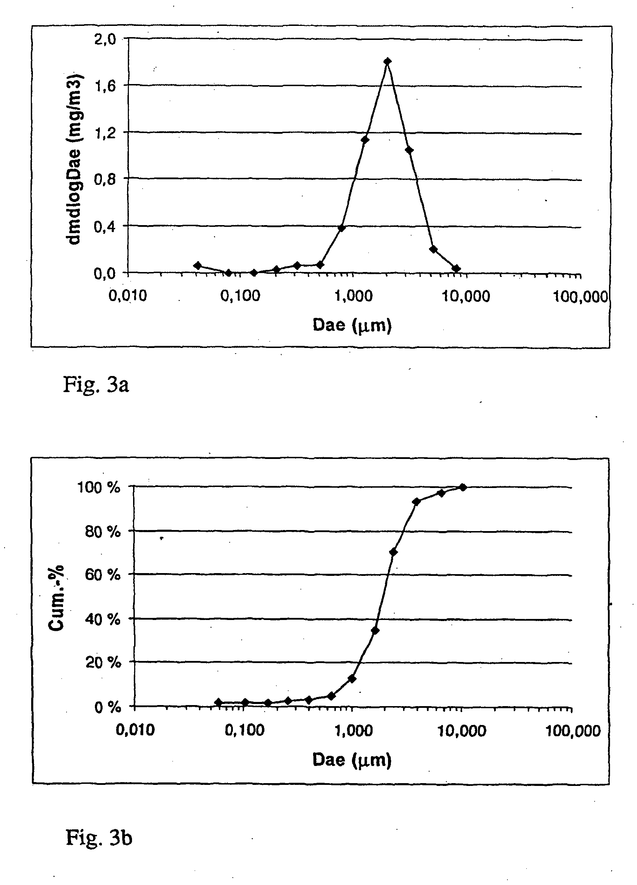

Referring now to Fig. 1b, the particle size distribution was measured by an electrical low pressure impactor (12) (ELPI) connected to a vacuum (13). The particles exiting the tubular tube were passed into a diluter (10), with a dilution ratio of 1:10, before entering the ELPI. Exhaust gas exit (11) was arranged in the diluter. To minimize temperature gradient, and thus to reduce the moisture condensation, the diluter, the line to the diluter and the gas line into the diluter were layered with heating elements, which were kept at a temperature higher than that of the solution dew point. Figures 3a and 3b show normalised and cumulative mass size distributions of beclomethasone dipropionate particles, respectively, measured gravimetrically. It is shown that a narrow size distribution within the range of aerodynamic particle size of interest, i.e. at around 1-5 µm, was obtained.

### ii. Particle crystallinity and polymorphism analysis

Crystallinity and polymorphism of the sample was studied by X-ray powder diffraction (Diffractometer D500, Siemens GmbH, Karlsruhe, Germany). A copper target X-ray (wavelength 0.1541nm) tube was operated with the power of 40 kV x 40 mA.

For x-ray powder diffraction analysis, 10 mg of the sample was mounted to a specific cylindrical *single crystal* mini sample stage, which has a diameter of 10 mm and height of approximately 1 mm.

The polymorphism of the powder was determined by comparing the diffraction pattern of the powder to that of a reference beclomethasone dipropionate powder supplied by Orion Corporation, Finland. The patterns obtained were identical. There were not significant differences in peak positions and additional peaks were not observed. Therefore the powder produced was classified as the previously known polymorphic crystal form class I, as expected.

The relative degree crystallinity of the powder was also determined based on the x-ray powder diffraction patterns. It is noticed that the powder was well crystallised. The maximum intensities were sharp and well above background intensities. The relative degree of crystallinity of reference and analysed powder are shown in Table 1. The estimation was based on the broadening of the diffraction maxima (FWHM-values) positioned at 11.3° and 18.4°. It is seen that the relative degree of crystallinity of the powder is 21 % higher than that of the reference powder.

**Table 1. Relative degree of crystallinity of reference and analysed powder.**

| Batch number | FWHMₐᵥ (°) | Relative degree of crystallinity (%) |
|---|---|---|
| Reference powder | 0.33 | 79 |
| Obtained beclomethasone dipropionate powder | 0.26 | 100 |

### iii. Particle shape and surface structure

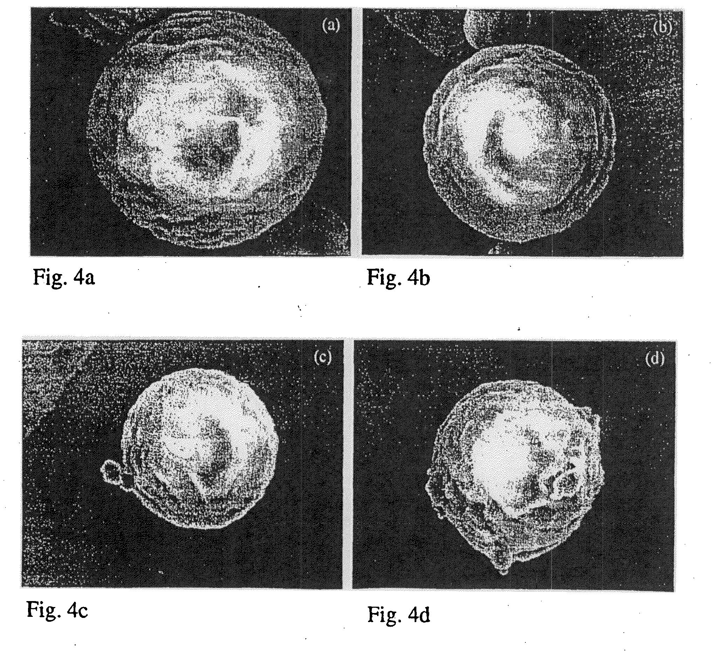

Referring now to Fig 1c, individual particles were collected on the surface of a holey carbon film TEM grid (14) connected to a vacuum (15) after particle collection. The morphology of the particles were then imaged using a field emission low voltage scanning electron microscope (FE-SEM) operated at 2 kV acceleration voltage. Figures 4a to 4d are scanning electron microscope images of the powder (magnification x20000 in Figs. 4b, 4c, 4d and x23000 in Fig. 4a). It is shown that the particles are spherical with rough surfaces and diameter of about 2-3 µm.

### iv. Chemical analysis

The product purity was analysed using Hewlett_Packard HP 1090 Liquid Chromatograph equipped with diode array detector. The column used is Hewlett-Packard Hypersil ODS, 5 µm, 100×2.1 mm. The powder analysed was dissolved into 10 ml of a mixture of water-ethanol (27:75) and then diluted (1:50) before the analysis. Samples were analysed with the high performance liquid chromatograph using diode array detector (wavelength 241 nm) and quantitative analysis was carried using external standard (six different standard concentrations). Eluents used were water (solvent A) and acetonitrile (solvent B) with gradient elution of 65% B for 2 minutes followed by 100%B in 5 minutes, and with flow rate of 0.4 ml/min. The oven temperature was set to 40°C. The analysis results showed that the purity of resulted powder is 100%.

### Example 2. Dry inhalation powder

| | Per dose |
|---|---|
| Beclomethasone dipropionate | 200 µg |
| Lactose monohydrate Ph. Eur. | 8 mg |

Particles of Example 1 and part of lactose is added into a blender. The powder mixture is mixed until it is homogenous. The mixture is sieved to reduce the number of particle clusters present. Thereafter the rest of lactose is added and the powder is again mixed until it is homogenous. Powder is poured into a supply chamber of the multi-dose powder inhaler Easyhaler (Orion Corporation trademark) for a supply of 200 doses.

## Claims

1. A method for preparing particles suitable for pulmonary drug delivery, comprising the steps of:
providing a liquid feed stock comprising an active agent;
atomising the liquid feed stock to create droplets;
suspending said droplets in a carrier gas;
passing said carrier gas and droplets suspended therein through a heated tube flow reactor under predetermined residence time and temperature history; and
collecting the particles produced.

2. A method of claim 1 wherein the liquid feed stock is in the form of a solution.

3. A method according to claim 1 or 2, wherein the carrier gas is selected from nitrogen gas or an inert gas.

4. A method according to claim 1, 2 or 3, wherein the particles are collected using a particle collection system selected from a group consisting of an electrostatic precipitator, a cyclone or a filter.

5. A method according to claim 4, wherein the particle collection system is heated to a temperature above the dew point temperature of the solution to prevent condensation.

6. A method according to any of claims 1 to 5, wherein the active agent is beclomethasone dipropionate.

7. A method according to claim 6, wherein the solvent is acetone, chloroform or ethanol.

8. A method according to any of claims 1 to 7, wherein the particles are of a predetermined polymorphic form.

## Patentansprüche

1. Verfahren zum Herstellen von Teilchen, die zur pulmonalen Arzneimittelabgabe geeignet sind, umfassend die folgenden Schritte:
Bereitstellen eines flüssigen Rohmaterials, das einen Wirkstoff umfasst;
Zerstäuben des flüssigen Rohmaterials, um Tröpfchen zu erzeugen;
Suspendieren der Tröpfchen in einem Trägergas;
Leiten des Trägergases und der darin suspendierten Tröpfchen durch einen beheizten Strömungsrohrreaktor unter einer vorbestimmten Verweilzeit und einem Temperaturverlauf; und
Sammeln der produzierten Teilchen.

2. Verfahren nach Anspruch 1, wobei das flüssige Rohmaterial in Form einer Lösung ist.

3. Verfahren nach Anspruch 1 oder 2, wobei das Trägergas aus Stickstoffgas oder einem Inertgas ausgewählt ist.

4. Verfahren nach Anspruch 1, 2 oder 3, wobei die Teilchen unter Verwendung eines Teilchensammelsystems gesammelt werden, das aus einer Gruppe bestehend aus einem Elektrofilter, einem Fliehkraftabscheider oder einem Filter ausgewählt ist.

5. Verfahren nach Anspruch 4, wobei das Teilchensammelsystem auf eine Temperatur über der Taupunkttemperatur der Lösung erwärmt wird, um eine Kondensation zu verhindern.

6. Verfahren nach einem der Ansprüche 1 bis 5, wobei der Wirkstoff Beclometasondipropionat ist.

7. Verfahren nach Anspruch 6, wobei das Lösungsmittel Aceton, Chloroform oder Ethanol ist.

8. Verfahren nach einem der Ansprüche 1 bis 7, wobei die Teilchen eine vorbestimmte polymorphe Form aufweisen.

## Revendications

1. Procédé de préparation de particules convenant à l'administration de médicament pulmonaire, comprenant les étapes de :
fourniture d'une charge d'alimentation liquide comprenant un agent actif ;
atomisation de la charge d'alimentation liquide pour créer des gouttelettes ;
suspension desdites gouttelettes dans un gaz vecteur ;
passage dudit gaz vecteur et de gouttelettes en suspension dans celui-ci par un réacteur à écoulement en tube chauffé avec un temps de séjour et un historique de température prédéterminés ; et
recueil des particules produites.

2. Procédé selon la revendication 1, dans lequel la charge d'alimentation liquide est sous forme d'une solution.

3. Procédé selon la revendication 1 ou 2, dans lequel le gaz vecteur est choisi parmi le gaz d'azote ou un gaz inerte.

4. Procédé selon la revendication 1, 2 ou 3, dans lequel les particules sont recueillies à l'aide d'un système de recueil de particules choisi dans un groupe constitué par un précipitateur électrostatique, un cyclone ou un filtre.

5. Procédé selon la revendication 4, dans lequel le système de recueil de particules est chauffé jusqu'à une température au-dessus de la température du point de rosée de la solution pour empêcher une condensation.

6. Procédé selon l'une quelconque des revendications 1 à 5, dans lequel l'agent actif est le dipropionate de béclométhasone.

7. Procédé selon la revendication 6, dans lequel le solvant est l'acétone, le chloroforme ou l'éthanol.

8. Procédé selon l'une quelconque des revendications 1 à 7, dans lequel les particules sont d'une forme polymorphe prédéterminée.
